# EUROPEAN PATENT SPECIFICATION

(11) **EP 3 731 936 B1**
(45) Date of publication and mention of the grant of the patent: **13.08.2025**
(21) Application number: 18826713.2
(22) Date of filing: 21.12.2018
(51) Int. Cl.: A61Q 5/12, A61Q 5/00, A61K 8/06, A61K 8/31, A61K 8/34, A61K 8/41, A61K 8/92

(54) **COSMETIC COMPOSITION IN THE FORM OF AN OIL-IN-WATER NANOEMULSION COMPRISING AT LEAST 40% OF FATTY SUBSTANCE, AT LEAST ONE CATIONIC SURFACTANT AND WATER**
KOSMETISCHE ZUSAMMENSETZUNG IN FORM EINER ÖL-IN-WASSER-NANOEMULSION MIT MINDESTENS 40 % FETTSUBSTANZ, MINDESTENS EINEM KATIONISCHEN TENSID UND WASSER
COMPOSITION COSMÉTIQUE SOUS FORME DE NANOÉMULSION HUILE-DANS-EAU COMPRENANT AU MOINS 40% DE CORPS GRAS, AU MOINS UN TENSIOACTIF CATIONIQUE ET DE L'EAU

(30) Priority: 29.12.2017 FR 1763395
(43) Date of publication of application: 04.11.2020
(73) Proprietor: L'OREAL, 75008 Paris (FR)
(72) Inventor: DION, Aurore, 93400 SAINT OUEN (FR); GABIN, Gérard, 93400 SAINT OUEN (FR)
(74) Representative: Casalonga
(86) International application number: PCT/EP2018/086567
(87) International publication number: WO 2019/129703

(56) References cited:
- EP-A1- 1 010 415
- EP-A1- 2 343 036
- EP-A2- 1 120 101
- EP-A2- 1 129 684
- WO-A1-2016/097387
- WO-A2-2008/051186
- FR-A1- 2 760 970
- ANONYMOUS: "Cetylpyridinium Chloride - an overview | ScienceDirect Topics", 31 December 2014 (2014-12-31), pages 1 - 9, XP093010363, Retrieved from the Internet <URL:https://www.sciencedirect.com/topics/chemistry/cetylpyridinium-chloride> [retrieved on 20221221]

## Description

The present invention relates to a cosmetic composition in the form of an oil-in-water nanoemulsion comprising (i) at least 40% by weight, relative to the total weight of the composition, of one or more fatty substances including at least one plant oil, (ii) one or more cationic surfactants, and (iii) water.

The present invention also relates to a process for the cosmetic treatment of keratin materials, in particular keratin fibres, preferably human keratin fibres such as the hair, in which the composition is applied to said materials.

Finally, the invention relates to the use of the composition for the cosmetic treatment of keratin materials, in particular keratin fibres, preferably human keratin fibres such as the hair.

Compositions in the form of nanoemulsions, in particular the oil-in-water type (O/W), are well known in the cosmetics and dermatology domain, in particular for preparing cosmetic products such as lotions, creams, tonics, serums or eau de toilette to be applied to the skin and/or hair.

Compositions in nanoemulsion form generally have a number-average oil drop (or oily globule) size less than 100 nanometres, said oil drops being stabilized by a layer of amphiphilic molecules that can optionally form a liquid crystal phase of the lamellar type, located at the oil/aqueous phase interface.

The term "amphiphilic molecule" is intended to mean herein any molecule having a bipolar structure, i.e. including at least one hydrophobic part and at least one hydrophilic part and having the property of reducing the surface tension of water (γ < 55mN/m) and of reducing the interface tension between water and an oily phase. Amphiphilic molecules are for example surfactants, surface agents or emulsifiers.

Due to the small oil drop size, compositions in the form of a nanoemulsion offer a certain number of advantages from a cosmetic or dermatological point of view leading in particular to a uniform and effective deposit of the composition on the skin and/or hair; in particular for the hair, a uniform deposit from the root to the end, while allowing satisfactory oil penetration and persistence.

Furthermore, compositions in nanoemulsion form confer improved cosmetic properties on keratin materials. In particular, for keratin fibres such as the hair, compositions in nanoemulsion form confer good disentangling, softness, feel, rinsability and suppleness properties and have a very good conditioning effect, which usually prove to be better than those obtained with the conventional emulsions and dispersions used in the cosmetics field.

EP 2 343 036 A1 discloses a cosmetic composition in the form of an oil-in-water nanoemulsion comprising one or more nonionic amphiphilic lipids, one or more volatile linear alkanes and one or more oils different from said volatile linear alkane(s).

Nevertheless, these compositions remain relatively liquid which can present disadvantages, for example in terms of usage qualities. More specifically, such compositions do not make it possible to guarantee sufficiently precise application to the fibres without the risk of running, in particular when they are sprayed.

In addition, there is still a need to obtain new innovative textures, in particular for compositions in nanoemulsion form, while at the same time retaining the advantages offered by this type of composition.

It has now been discovered, surprisingly, that a composition in the form of a nanoemulsion comprising (i) at least 40% by weight, relative to the total weight of the composition, of one or more fatty substances including at least one plant oil, (ii) one or more cationic surfactants, and (iii) water make it possible to meet the needs expressed above.

In particular, such a composition has an original gellified texture which does not require the addition of thickening polymers or a gelling agent.

A subject of the invention is thus a cosmetic composition in the form of an oil-in-water nanoemulsion comprising:
(i) at least 40% by weight, relative to the total weight of the composition, of one or more fatty substances including at least one plant oil,
(ii) one or more cationic surfactants chosen from:
   - those corresponding to general formula (I) below: 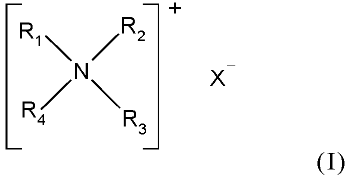 in which the groups R₁ to R₄, which may be identical or different, represent a linear or branched aliphatic group comprising from 1 to 30 carbon atoms, at least one of the groups R₁ to R₄ denoting a linear or branched aliphatic radical comprising from 8 to 30 carbon atoms, preferably from 12 to 24 carbon atoms,
      X⁻ is an anion chosen from the group of halides, phosphates, acetates, lactates, (C₁-C₄)alkyl sulfates, (C₁-C₄)alkylsulfonates or (C₁-C₄)alkylarylsulfonates,
   - quaternary ammonium salts of imidazoline,
   - di- or tri-quaternary ammonium salts,
   - quaternary ammonium salts containing at least one ester function, and
(iii) water.

The composition according to the invention thus has a gelled texture ensuring a certain number of advantages.

First of all, the composition according to the invention has an attractive visual for the consumer.

In addition, it has a creamy texture which in particular makes it possible in particular to achieve a better localization of the composition on the keratin materials, in particular the keratin fibres such as the hair, during its application while at the same time minimizing the risks of running and/or splashing, in particular into the eyes.

In addition, the composition according to the invention enables a gentle and uniform coating of the keratin fibres from the root to the end.

The depositing of active products on the keratin fibres (fatty substances, vitamins) also proves to be greater than for a conventional care product.

In other words, the composition has improved usage qualities guaranteeing both a distribution without running that is more targeted at the point of application, and uniform spreading on the keratin fibres.

Moreover, the composition according to the invention is stable with respect to storage over time, both at ambient temperature (25°C) and at higher temperatures, in particular 45°C.

For the purposes of the present invention, the term "stable with respect to storage over time" is intended to mean that the following physical characteristics of the composition vary little, or even not at all, over time: appearance, pH, rheology (viscosity, consistency).

In particular, the composition does not give rise to any phase-separation or exudation phenomena over time, and at the intended storage temperature.

The composition above also confers improved cosmetic properties on the keratin materials treated with these compositions. In particular for keratin fibres, preferably the hair, the composition according to the invention confers good rinsability properties, and good conditioning properties, in particular in terms of disentangling, softness, feel and suppleness.

More specifically, the composition according to the invention, on contact with the keratin materials, is converted into a bluish-coloured milk, which makes it very easy and quick to rinse off.

Finally, the composition above has the advantage of being able to be prepared by any type of preparation process, and in particular using a process which is low in energy and easily industrializable.

The invention also relates to a process for the cosmetic treatment of keratin materials, in particular human keratin fibres such as the hair, in which the composition according to the invention is applied to said materials.

Finally, the invention relates to the use of said composition for the cosmetic treatment of keratin materials, in particular human keratin fibres such as the hair.

Likewise, the composition according to the invention can also be used for caring for the skin.

Other subjects, characteristics, aspects and advantages of the invention will emerge even more clearly on reading the description and the examples that follow.

In the following text, and unless otherwise indicated, the limits of a range of values are included in that range, especially in the expressions "between" and "ranging from ... to ...".

Moreover, the expression "at least one" used in the present description is equivalent to the expression "one or more".

The cosmetic composition according to the invention is preferably a cosmetic composition for treating keratin fibres, in particular human keratin fibres such as the hair.

The composition according to the invention comprises at least 40% by weight, relative to the total weight of the composition, of one or more fatty substances, including at least one plant oil.

The term "fatty substance" is intended to mean an organic compound that is insoluble in water at ambient temperature (25°C) and at atmospheric pressure (1.013×10⁵ Pa), i.e. which has a solubility of less than 5% by weight, preferably less than 1% by weight. They are generally soluble, under the same temperature and pressure conditions, in organic solvents such as chloroform, ethanol, benzene, liquid petroleum jelly or decamethylcyclopentasiloxane.

Preferably, the fatty substance(s) are chosen from fatty substances that are liquid at 30°C and at atmospheric pressure (1.013×10⁵ Pa).

Preferably, the fatty substance(s) of the composition according to the invention are non-silicone.

The term "non-silicone fatty substance" is intended to mean a fatty substance of which the structure does not comprise any silicon atoms, and which therefore especially does not comprise any siloxane groups. They generally bear in their structure a hydrocarbon-based chain comprising at least 6 carbon atoms. Advantageously, they are not oxyalkylenated and do not contain any -COOH functions.

The fatty substances that can be used in the invention preferably have a viscosity of less than or equal to 2 Pa.s, better still less than or equal to 1 Pa.s and even better still less than or equal to 0.1 Pa.s at a temperature of 30°C and at a shear rate of 1 s⁻¹ measured with a Rheomat RM1800 (generally with spindle 1 or 2).

The fatty substances that can be used in the composition according to the invention may be chosen especially from liquid hydrocarbons, liquid fatty alcohols, liquid fatty esters including plant oils, and liquid fatty acids, and mixtures of these compounds.

For the purposes of the present invention, the term "liquid hydrocarbon" is intended to mean a hydrocarbon composed solely of carbon and hydrogen atoms, which is liquid at a temperature of 30°C and at atmospheric pressure (760 mmHg, i.e. 1.013×10⁵ Pa).

More particularly, the liquid hydrocarbons are chosen from:
- linear or branched, optionally cyclic, C₆-C₁₆ alkanes. Examples that may be mentioned include hexane, undecane, dodecane, tridecane, and isoparaffins, for instance isohexadecane, isododecane and isodecane;
- linear or branched hydrocarbons of mineral, animal or synthetic origin with more than 16 carbon atoms, such as liquid paraffins and derivatives thereof, petroleum jelly, liquid petroleum jelly, polydecenes, hydrogenated polyisobutene such as the product sold under the brand name Parleam^{®} by the company NOF Corporation, and squalane.

Preferably, the liquid hydrocarbon(s) are chosen from liquid paraffins, isoparaffins, liquid petroleum jelly, undecane, tridecane and isododecane, and mixtures thereof.

**In** a most particularly preferred variant, the liquid hydrocarbon(s) are chosen from liquid petroleum jelly, isoparaffins, isododecane and a mixture of undecane and tridecane.

For the purposes of the present invention, the term "liquid fatty alcohol" is intended to mean a non-glycerolated and non-oxyalkylenated fatty alcohol, which is liquid at a temperature of 30°C and at atmospheric pressure (760 mmHg, i.e. 1.013×10⁵ Pa). Preferably, the liquid fatty alcohols of the invention comprise from 8 to 30 carbon atoms, especially from 10 to 24 carbon atoms, and may be saturated or unsaturated.

The saturated liquid fatty alcohols are preferably branched. They may optionally comprise in their structure at least one aromatic or non-aromatic ring, which is preferably acyclic.

More particularly, the saturated liquid fatty alcohols of the invention are chosen from octyldodecanol, 2-decyltetradecanol, isostearyl alcohol and 2-hexyldecanol.

Octyldodecanol and 2-decyltetradecanol are most particularly preferred.

The unsaturated liquid fatty alcohols contain in their structure at least one double or triple bond, and preferably one or more double bonds. When several double bonds are present, there are preferably 2 or 3 of them, and they may be conjugated or unconjugated.

These unsaturated fatty alcohols may be linear or branched.

They may optionally comprise in their structure at least one aromatic or non-aromatic ring. They are preferably acyclic.

More particularly, the unsaturated liquid fatty alcohols that may be used in the invention are chosen from oleyl alcohol, linoleyl alcohol, linolenyl alcohol and undecylenyl alcohol.

Oleyl alcohol is most particularly preferred.

For the purposes of the present invention, the term "liquid fatty ester" is intended to mean an ester derived from a fatty acid and/or from a fatty alcohol, which is liquid at a temperature of 30°C and at atmospheric pressure (760 mmHg, i.e. 1.013×10⁵ Pa).

More particularly, the liquid esters are chosen from esters of saturated or unsaturated, linear or branched C₁-C₂₆ aliphatic mono- or polyacids, which are optionally hydroxylated, and from saturated or unsaturated, linear or branched C₁-C₂₆ aliphatic mono- or polyalcohols, the total number of carbon atoms in the esters being greater than or equal to 10.

Preferably, for the esters of monoalcohols, at least one from among the alcohol and the acid from which the esters of the invention are derived is branched.

Among the monoesters of monoacids and of monoalcohols, mention may be made of alkyl palmitates, in particular of C₁-C₁₈, especially ethyl palmitate and isopropyl palmitate, alkyl myristates in particular of C₁-C₁₈, such as isopropyl myristate or ethyl myristate, alkyl stearates, in particular of C₁-C₁₈, especially isocetyl stearate, 2-ethylhexyl isononanoate, isodecyl neopentanoate and isostearyl neopentanoate.

Esters of optionally hydroxylated C₃-C₂₂ dicarboxylic or tricarboxylic acids and of C₁-C₂₂ alcohols and esters of optionally hydroxylated monocarboxylic, dicarboxylic or tricarboxylic acids and of C₄-C₂₆ dihydroxy, trihydroxy, tetrahydroxy or pentahydroxy non-sugar alcohols may also be used.

Mention may be made especially of diethyl sebacate, diisopropyl sebacate, diisopropyl adipate, di-n-propyl adipate, dioctyl adipate, diisostearyl adipate, dioctyl maleate, glyceryl undecylenate, octyldodecyl stearoyl stearate, pentaerythrityl monoricinoleate, pentaerythrityl tetraisononanoate, pentaerythrityl tetrapelargonate, pentaerythrityl tetraisostearate, pentaerythrityl tetraoctanoate, propylene glycol dicaprylate, propylene glycol dicaprate, tridecyl erucate, triisopropyl citrate, triisostearyl citrate, glyceryl trilactate, glyceryl trioctanoate, trioctyldodecyl citrate, trioleyl citrate, propylene glycol dioctanoate, neopentyl glycol diheptanoate, diethylene glycol diisononanoate, polyethylene glycol distearates, and alkyl malates, especially (C₆-C₁₈)alkyl malates, in particular bis(C₁₂-C₁₃)alkyl malate. Among the esters mentioned above, use is preferentially made of ethyl, isopropyl, myristyl, cetyl or stearyl palmitate, 2-ethylhexyl palmitate, 2-octyldecyl palmitate, alkyl myristates such as isopropyl, butyl, cetyl or 2-octyldodecyl myristate, hexyl stearate, propylene glycol dicaprylate, butyl stearate, isobutyl stearate; dioctyl malate, hexyl laurate, 2-hexyldecyl laurate, isononyl isononanoate, cetyl octanoate and bis(C₁₂-C₁₃)alkyl malate. Among the liquid fatty esters, use may be made of sugar esters and diesters of C₆-C₃₀ and preferably C₁₂-C₂₂ fatty acids.

The term "sugar" is intended to mean oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Preferably, these said sugars are chosen from sucrose, glucose, galactose, ribose, fucose, maltose, fructose, mannose, arabinose, xylose and lactose, and derivatives thereof, especially alkyl derivatives, such as methyl derivatives, for instance methylglucose.

The sugar and fatty acid esters may be chosen especially from the group comprising the esters or mixtures of esters of sugars described previously and of linear or branched, saturated or unsaturated C₆-C₃₀ and preferably C₁₂-C₂₂ fatty acids.

If they are unsaturated, these compounds may comprise one to three conjugated or unconjugated carbon-carbon double bonds.

The esters according to this variant may also be chosen from mono-, di-, tri- and tetraesters, and polyesters, and mixtures thereof.

These esters may be, for example, oleates, laurates, palmitates, myristates, behenates, cocoates, stearates, linoleates, linolenates, caprates and arachidonates, and mixtures thereof, such as, especially, oleopalmitate, oleostearate or palmitostearate mixed esters.

More particularly, use is made of monoesters and diesters and especially of sucrose, glucose or methylglucose mono- or dioleates, stearates, behenates, oleopalmitates, linoleates, linolenates or oleostearates, or alternatively of methylglucose dioleate (Glucate^{®} DO).

Among the sugar esters, use may be made of pentaerythrityl esters, preferably pentaerythrityl tetraisostearate, pentaerythrityl tetraoctanoate, and caprylic and capric acid hexaesters as a mixture with dipentaerythritol.

Among the natural or synthetic monoacid, diacid or triacid esters with glycerol, use may be made of plant oils or synthetic oils.

More particularly, said plant oil(s) or synthetic oil(s) are chosen from triglyceride oils of plant or synthetic origin, such as liquid fatty acid triglycerides containing from 6 to 30 carbon atoms, for instance heptanoic or octanoic acid triglycerides, or alternatively, for example, sesame oil, soybean oil, coffee oil, safflower oil, borage oil, sunflower oil, olive oil, apricot kernel oil, camellia oil, bambara pea oil, avocado oil, mango oil, rice bran oil, cottonseed oil, rose oil, kiwi seed oil, sea buckthorn pulp oil, blueberry seed oil, poppy seed oil, orange pip oil, sweet almond oil, palm oil, copra oil, vernonia oil, marjoram oil, baobab oil, rapeseed oil, ximenia oil, pracaxi oil, caprylic/capric acid triglycerides such as those sold by the company Stéarineries Dubois or those sold under the names Miglyol^{®} 810, 812 and 818 by the company Dynamit Nobel, jojoba oil and shea butter oil.

As liquid esters that may be used according to the invention, use is preferably made of triglycerides of plant origin, in particular oils chosen from avocado oil, olive oil, camellia oil and apricot kernel oil, sunflower oil, shea oil, jojoba oil, soybean oil, copra (or coconut) oil, rapeseed oil, and mixtures thereof, and C₄-C₂₂ dicarboxylic or tricarboxylic acid esters of C₁-C₂₂ alcohols, in particular 1,3-propanediol dicaprylate.

The term "fatty acid" is intended to mean a non-salified fatty acid, i.e. the fatty acid must not be in the form of a generally soluble soap, i.e. it must not be salified with a base.

More particularly, the liquid fatty acids that may be used according to the invention are chosen from the acids of formula RCOOH, in which R is a saturated or unsaturated, linear or branched radical preferably comprising from 7 to 39 carbon atoms.

Preferably, R is a C₇-C₂₉ alkyl or C₇-C₂₉ alkenyl group and better still a C₁₂-C₂₄ alkyl or C₁₂-C₂₄ alkenyl group. R may be substituted with one or more hydroxyl groups and/or one or more carboxyl groups.

Preferentially, the liquid fatty acid(s) are chosen from oleic acid, linoleic acid and isostearic acid.

As indicated above, the composition according to the invention comprises at least one plant oil, preferably the plant oil(s) is(are) chosen from one or more triglyceride oils, and in particular chosen from sesame oil, soybean oil, coffee oil, safflower oil, borage oil, sunflower oil, olive oil, apricot kernel oil, camelia oil, bambara pea oil, avocado oil, mango oil, rice bran oil, cottonseed oil, rose oil, kiwi seed oil, sea buckthorn pulp oil, blueberry seed oil, poppy seed oil, orange pip oil, sweet almond oil, palm oil, copra oil, vernonia oil, marjoram oil, baobab oil, rapeseed oil, ximenia oil, pracaxi oil, jojoba oil, shea butter oil and mixtures thereof.

Preferentially, the composition according to the invention comprises one or more plant oils chosen from avocado oil, olive oil, camelia oil, apricot kernel oil, sunflower oil, shea oil, jojoba oil, soybean oil, copra (or coconut) oil, rapeseed oil and mixtures thereof.

The composition may also comprise one or more additional fatty substances other than plant oils, preferably chosen from liquid fatty substances other than plant oils, preferentially chosen from liquid hydrocarbons, liquid fatty esters other than plant oils and mixtures of these compounds.

Most particularly preferably, the composition according to the invention comprises one or more additional, preferably liquid, fatty substances chosen from linear or branched C₆-C₁₆ alkanes, and in particular chosen from undecane, tridecane, and a mixture of these compounds.

Most particularly preferably, the composition according to the invention comprises, as fatty substance:
- one or more plant oils, in particular chosen from triglyceride oils, preferentially from avocado oil, olive oil, camelia oil, apricot kernel oil, sunflower oil, shea oil, jojoba oil, soybean oil, copra (or coconut) oil, rapeseed oil and mixtures thereof; and
- one or more additional, preferably liquid, fatty substances other than plant oils, better still chosen from linear or branched C₆-C₁₆ alkanes, and in particular chosen from undecane, tridecane, and mixtures thereof.

The composition according to the invention comprises one or more, preferably liquid, fatty substances, including at least one plant oil generally in a total content ranging from 42% to 90% by weight, preferably from 43% to 85% by weight and more preferentially from 45% to 80% by weight, better still from 48% to 70% by weight, relative to the total weight of the composition.

According to one preferred variant of the invention, the composition of the invention comprises at least 30% by weight of one or more plant oils, preferably at least 40% by weight, relative to the total weight of the composition.

In particular, the composition of the invention comprises the plant oil(s) in a total content ranging from 30% to 90% by weight, preferably from 35% to 85% by weight, more preferentially from 40% to 70% by weight, relative to the total weight of the composition.

The composition comprises one or more cationic surfactants chosen from those corresponding to general formula (I) as described below, quaternary ammonium salts of imidazoline, di- or tri-quaternary ammonium salts and quaternary ammonium salts containing at least one ester function.

The term "cationic surfactant" is intended to mean a surfactant that is positively charged when it is contained in the composition according to the invention. This surfactant may bear one or more positive permanent charges or may contain one or more cationizable functions in the composition according to the invention.

The cationic surfactants which may be used in the composition according to the invention are:
- those corresponding to general formula (I) below: 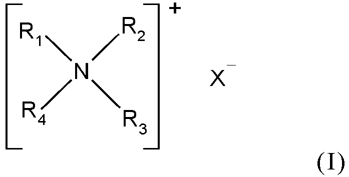 in which the groups R₁ to R₄, which may be identical or different, represent a linear or branched aliphatic group comprising from 1 to 30 carbon atoms, at least one of the groups R₁ to R₄ denoting a linear or branched aliphatic radical comprising from 8 to 30 carbon atoms, preferably from 12 to 24 carbon atoms. The aliphatic groups may comprise heteroatoms especially such as oxygen, nitrogen, sulfur and halogens. The aliphatic groups are chosen, for example, from C₁-C₃₀ alkyl, C₁-C₃₀ alkoxy, polyoxy(C₂-C₆)alkylene, C₁-C₃₀ alkylamide, (C₁₂-C₂₂)alkylamido(C₂-C₆)alkyl, (C₁₂-C₂₂)alkyl acetate and C₁-C₃₀ hydroxyalkyl groups; X⁻ is an anion chosen from the group of the halides, phosphates, acetates, lactates, (C₁-C₄)alkyl sulfates and (C₁-C₄)alkyl- or (C₁-C₄)alkylarylsulfonates.

Among the quaternary ammonium salts of formula (I), the ones that are preferred are, on the one hand, tetraalkylammonium salts, for instance dialkyldimethylammonium or alkyltrimethylammonium salts in which the alkyl group comprises approximately from 12 to 22 carbon atoms, in particular behenyltrimethylammonium, distearyldimethylammonium or cetyltrimethylammonium salts, or, on the other hand, the palmitylamidopropyltrimethylammonium salts, the stearamidopropyltrimethylammonium salts, the stearamidopropyldimethylcetearylammonium salts, or the stearamidopropyldimethyl(myristyl acetate)ammonium salts sold under the name Ceraphyl^{®} 70 by the company Van Dyk. It is preferred in particular to use the chloride salts of these compounds.
- quaternary ammonium salts of imidazoline, for instance those of formula (II) below: 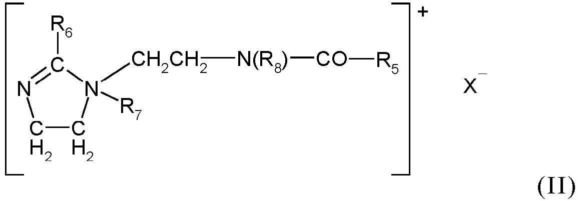 in which R₅ represents an alkenyl or alkyl group comprising from 8 to 30 carbon atoms, for example derived from tallow fatty acids, R₆ represents a hydrogen atom, a C₁-C₄ alkyl group or an alkenyl or alkyl group comprising from 8 to 30 carbon atoms, R₇ represents a C₁-C₄ alkyl group, R₈ represents a hydrogen atom, a C₁-C₄ alkyl group and X⁻ is an anion chosen from the group of the halides, phosphates, acetates, lactates, alkyl sulfates, alkylsulfonates or alkylarylsulfonates, the alkyl and aryl groups of which preferably comprise, respectively, from 1 to 20 carbon atoms and from 6 to 30 carbon atoms. Preferably, R₅ and R₆ denote a mixture of alkenyl or alkyl groups comprising from 12 to 21 carbon atoms, for example derived from tallow fatty acids, R₇ denotes a methyl group and R₈ denotes a hydrogen atom. Such a product is sold, for example, under the name Rewoquat^{®} W 75 by the company Rewo;
- di- or tri-quaternary ammonium salts, in particular of formula (III): 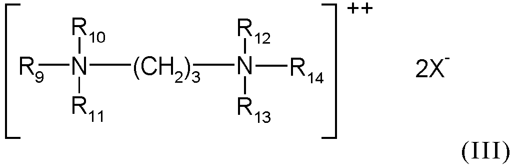 in which R₉ denotes an alkyl radical comprising approximately from 16 to 30 carbon atoms, which is optionally hydroxylated and/or optionally interrupted with one or more oxygen atoms, R₁₀ is chosen from hydrogen or an alkyl radical comprising from 1 to 4 carbon atoms or a group (R₉ₐ)(R₁₀ₐ)(R₁₁ₐ)N-(CH₂)₃,
   R₉ₐ, R₁₀ₐ, R₁₁ₐ, R₁₁, R₁₂, R₁₃ and R₁₄, which may be identical or different, are chosen from hydrogen or an alkyl radical comprising from 1 to 4 carbon atoms, and X⁻ is an anion chosen from the group of halides, acetates, phosphates, nitrates, (C₁-C₄)alkyl sulfates, (C₁-C₄)alkyl sulfonates and (C₁-C₄)alkylaryl sulfonates, and in particular methyl sulfate and ethyl sulfate. Such compounds are, for example, Finquat CT-P, sold by the company Finetex (Quaternium 89), and Finquat CT, sold by the company Finetex (Quaternium 75),
- quaternary ammonium salts containing at least one ester function, such as those of formula (IV) below: 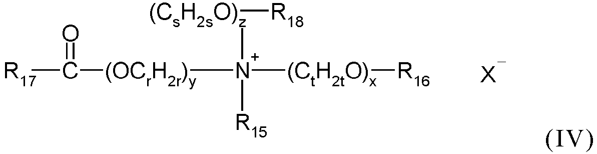 in which:
   R₁₅ is chosen from C₁-C₆ alkyl groups and C₁-C₆ hydroxyalkyl or dihydroxyalkyl groups;
   R₁₆ is chosen from:
      - the group 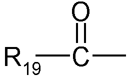
      - groups R₂₀, which are linear or branched, saturated or unsaturated C₁-C₂₂ hydrocarbon-based groups,
      - a hydrogen atom,
   R₁₈ is chosen from:
      - the group 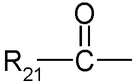
      - groups R₂₂, which are linear or branched, saturated or unsaturated C₁-C₆ hydrocarbon-based groups,
      - a hydrogen atom,
   R₁₇, R₁₉ and R₂₁, which may be identical or different, are chosen from linear or branched, saturated or unsaturated C₇-C₂₁ hydrocarbon-based groups;
   r, s and t, which may be identical or different, are integers ranging from 2 to 6;
   y is an integer ranging from 1 to 10;
   x and z, which may be identical or different, are integers ranging from 0 to 10;
   X⁻ is a simple or complex, organic or mineral, anion;
   with the proviso that the sum x + y + z is from 1 to 15, that when x is 0 then R₁₆ denotes R₂₀, and that when z is 0 then R₁₈ denotes R₂₂.

The alkyl groups R₁₅ may be linear or branched, and more particularly linear.

Preferably, R₁₅ denotes a methyl, ethyl, hydroxyethyl or dihydroxypropyl group, and more particularly a methyl or ethyl group.

Advantageously, the sum x + y + z is from 1 to 10.

When R₁₆ is a hydrocarbon-based group R₂₀, it may be long and contain from 12 to 22 carbon atoms, or may be short and contain from 1 to 3 carbon atoms.

When R₁₈ is a hydrocarbon-based group R₂₂, it preferably contains 1 to 3 carbon atoms.

Advantageously, R₁₇, R₁₉ and R₂₁, which may be identical or different, are chosen from linear or branched, saturated or unsaturated C₁₁-C₂₁ hydrocarbon-based groups, and more particularly from linear or branched, saturated or unsaturated C₁₁-C₂₁ alkyl and alkenyl groups.

Preferably, x and z, which may be identical or different, are equal to 0 or 1.

Advantageously, y is equal to 1.

Preferably, r, s and t, which may be identical or different, are equal to 2 or 3, and even more particularly are equal to 2.

The anion X⁻ is preferably a halide (chloride, bromide or iodide) or an alkyl sulfate, more particularly methyl sulfate. However, use may be made of methanesulfonate, phosphate, nitrate, tosylate, an anion derived from an organic acid, such as acetate or lactate, or any other anion that is compatible with the ammonium bearing an ester function.

The anion X⁻ is even more particularly chloride or methyl sulfate.

Use may be made more particularly in the composition according to the invention of the ammonium salts of formula (IV) in which:
R₁₅ denotes a methyl or ethyl group,
x and y are equal to 1;
z is equal to 0 or 1;
r, s and t are equal to 2;
R₁₆ is chosen from:
   - the group 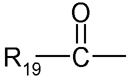
   - methyl, ethyl or C₁₄-C₂₂ hydrocarbon-based groups,
   - a hydrogen atom;
R₁₈ is chosen from:
   - the group 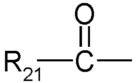
   - a hydrogen atom;
R₁₇, R₁₉ and R₂₁, which may be identical or different, are chosen from linear or branched, saturated or unsaturated C₁₃-C₁₇ hydrocarbon-based groups, and preferably from linear or branched, saturated or unsaturated C₁₃-C₁₇ alkyl and alkenyl groups.

Advantageously, the hydrocarbon-based groups are linear.

Examples that may be mentioned include the compounds of formula (IV) such as the diacyloxyethyldimethylammonium, diacyloxyethylhydroxyethylmethylammonium, monoacyloxyethyl-dihydroxyethylmethylammonium, triacyloxyethylmethylammonium and monoacyloxyethylhydroxyethyldimethylammonium salts (chloride or methyl sulfate in particular), and mixtures thereof. The acyl groups preferably contain from 14 to 18 carbon atoms and are derived more particularly from a plant oil such as palm oil or sunflower oil. When the compound contains several acyl groups, these groups may be identical or different.

These products are obtained, for example, by direct esterification of triethanolamine, triisopropanolamine, an alkyldiethanolamine or an alkyldiisopropanolamine, which are optionally oxyalkylenated, with C₁₀-C₃₀ fatty acids or with mixtures of C₁₀-C₃₀ fatty acids of plant or animal origin, or by transesterification of the methyl esters thereof. This esterification is followed by a quaternization using an alkylating agent such as an alkyl halide (preferably a methyl or ethyl halide), a dialkyl sulfate (preferably a methyl or ethyl sulfate), methyl methanesulfonate, methyl para-toluenesulfonate, glycol chlorohydrin or glycerol chlorohydrin.

Such compounds are sold, for example, under the names Dehyquart^{®} by the company Henkel, Stepanquat^{®} by the company Stepan, Noxamium^{®} by the company Ceca or Rewoquat^{®} WE 18 by the company Rewo-Witco.

The composition according to the invention may contain, for example, a mixture of quaternary ammonium monoester, diester and triester salts with a weight majority of diester salts.

Use may also be made of the ammonium salts containing at least one ester function that are described in patents US-A-4 874 554 and US-A-4 137 180.

Use may be made of behenoylhydroxypropyltrimethyl-ammonium chloride sold by KAO under the name Quatarmin BTC 131.

Preferably, the ammonium salts containing at least one ester function contain two ester functions.

Among the quaternary ammonium salts containing at least one ester function, which may be used, it is preferred to use dipalmitoyl-ethylhydroxyethylmethylammonium salts.

The cationic surfactant(s) are preferably chosen from those of formula (I) and those of formula (IV) and even more preferentially from those of formula (I).

Most particularly preferably, the cationic surfactant(s) of the invention are chosen from those of formula (I), more preferentially from behenyltrimethylammonium salts, cetyltrimethylammonium salts, and a mixture of these compounds, and even more preferentially from behenyltrimethylammonium chloride, cetyltrimethylammonium chloride, and a mixture of these compounds.

The cationic surfactant(s) advantageously represent from 0.01% to 20% by weight, preferably from 0.1% to 10% by weight and more preferentially from 0.1% to 8% by weight, relative to the total weight of the composition.

Particularly preferably, the composition according to the invention does not comprise any gelling agent, thickening polymers, fatty alcohols that are solid and fatty acids that are solid at a temperature of 30°C and at atmospheric pressure.

The composition according to the invention may also comprise one or more polyols.

For the purposes of the present invention, the term "polyol" is intended to mean an organic compound constituted of a hydrocarbon-based chain optionally interrupted with one or more oxygen atoms and bearing at least two free hydroxyl groups (-OH) borne by different carbon atoms, this compound possibly being cyclic or acyclic, linear or branched, and saturated or unsaturated.

More particularly, the polyol(s) that may be used according to the invention comprise from 2 to 30 hydroxyl groups, more preferentially from 2 to 10 hydroxyl groups and even more preferentially from 2 to 3 hydroxyl groups.

The polyol(s) that may be used according to the invention generally comprise at least three carbon atoms.

Preferably, said polyol(s) that may be used according to the invention are chosen from polyols comprising at least three carbon atoms and ethylene glycol, and are preferably chosen from 1,3-propanediol, 1,3-butylene glycol, 1,2-pentanediol, dipropylene glycol, hexylene glycol, pentylene glycol, glycerol, ethylene glycol and sorbitol, and a mixture of these compounds.

Most particularly preferably, the polyol(s) that can be used according to the invention are chosen from glycerol, sorbitol or a mixture of these compounds, and preferably the polyol is glycerol.

Advantageously, the composition according to the invention does not comprise propylene glycol.

When they are present, the polyol(s) represent from 1% to 20% by weight, better still from 2% to 15% by weight and more preferentially from 3% to 10% by weight, relative to the total weight of the composition.

The composition according to the invention generally comprises water.

Water advantageously represents from 10% to 80% by weight, preferably from 15% to 70% by weight and more preferentially from 20% to 65% by weight relative to the total weight of the composition.

The composition according to the invention may also comprise one or more organic solvents other than polyols.

Preferably, the organic solvent(s) other than polyols are chosen from non-aromatic C₁-C₆ alcohols such as ethyl alcohol or isopropyl alcohol, or aromatic alcohols such as benzyl alcohol and phenylethyl alcohol.

Particularly preferably, the composition according to the invention comprises one or more organic solvents other than polyols, the organic solvent preferably being ethanol.

When they are present in the composition according to the invention, the organic solvent(s) other than polyols generally represent from 0.1% to 15% by weight and preferably from 0.5% to 10% by weight relative to the total weight of the composition.

The pH of the composition of the invention is generally between 3 and 8, preferably between 4 and 7, better still between 5 and 6.

The pH of the composition according to the invention may be adjusted to the desired value by means of acidifying or basifying agents usually used in cosmetic compositions, or alternatively using standard buffer systems.

Among the acidifying agents, examples that may be mentioned include mineral acids, for instance hydrochloric acid, (ortho)phosphoric acid, boric acid, nitric acid or sulfuric acid, or organic acids, for instance compounds comprising at least one carboxylic acid function such as acetic acid, tartaric acid, citric acid or lactic acid, a sulfonic acid function, a phosphonic acid function or a phosphoric acid function.

Preferably, phosphoric acid is used as acidifying agent.

The basifying agent(s) may be mineral, organic or hybrid.

The mineral alkaline agent(s) are preferably chosen from aqueous ammonia, alkali metal carbonates or bicarbonates such as sodium carbonate or bicarbonate, potassium carbonate or bicarbonate, sodium hydroxide or potassium hydroxide or mixtures thereof.

The organic alkaline agent(s) are preferably chosen from organic amines with a pK_{b} at 25°C of less than 12, preferably of less than 10 and even more advantageously of less than 6. It should be noted that it is the pK_{b} corresponding to the function of highest basicity. In addition, the organic amines do not comprise any alkyl or alkenyl fatty chain comprising more than ten carbon atoms.

The organic alkaline agent(s) are chosen, for example, from alkanolamines, oxyethylenated and/or oxypropylenated ethylenediamines, amino acids and the compounds of formula (VIII) below: 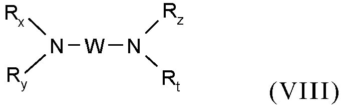 in which W is a divalent C₁ to C₆ alkylene radical optionally substituted with one or more hydroxyl groups or a C₁ to C₆ alkyl radical, and/or optionally interrupted with one or more heteroatoms such as O, or NRᵤ; Rₓ, R_{y}, R_{z}, Rₜ and Rᵤ, which may be identical or different, represent a hydrogen atom or a C₁ to C₆ alkyl, C₁ to C₆ hydroxyalkyl or C₁ to C₆ aminoalkyl radical.

Examples of amines of formula (VIII) that may be mentioned include 1,3-diaminopropane, 1,3-diamino-2-propanol, spermine and spermidine.

The composition according to the invention may also comprise one or more additives.

As additives that may be used in accordance with the invention, mention may be made of antidandruff agents, anti-seborrhoea agents, agents for preventing hair loss and/or for promoting hair regrowth, vitamins and provitamins including panthenol, sunscreens, mineral or organic pigments, sequestrants, plasticizers, solubilizers, opacifiers or pearlescent agents, antioxidants, fragrances, preservatives and pigments.

Those skilled in the art will take care to select the optional additives and the amount thereof such that they do not harm the properties of the composition according to the invention.

These additives may be present in the composition according to the invention in an amount ranging from 0 to 50% by weight, relative to the total weight of the composition.

The composition according to the invention is in the form of an oil-in-water nanoemulsion of which the particles (or drops) of oil preferably have a volume-average size of less than 500 nm, preferentially of between 10 and 300 nm, and better still between 20 and 250 nm, and even better still between 25 and 200 nm.

The volume-average size of the particles (or oil drops) may be determined in particular according to the known laser diffraction method. By way of apparatus that can be used for this determination, mention may be made of the particle size analyser of the brand Malvern, model Zetasizer Nano ZS, equipped with a standard laser having a power of 4 mW, and at a wavelength of 633 nm. This device is also equipped with a correlator (25 ns to 8000 s, 4000 channels maxi.).

In addition, the composition according to the invention has very low polydispersity, i.e. the particles have a very homogeneous size. The particles present in the composition according to the invention are liquid oil (or oily phase) particles within the continuous aqueous phase.

The composition according to the invention is advantageously in gel form.

Preferably, the composition has a translucent to blueish colour, preferably a blueish colour.

The composition according to the invention may have a viscosity ranging for example from 1 to 500 mPa.s (1 to 500 cPoises), and preferably from 1 to 200 mPa.s (1 to 200 cPoises), the viscosity being measured at ambient temperature (25°C) with a Rheomat RM180 (generally using spindle 1 or 2).

The invention also relates to a process for the cosmetic treatment of keratin materials, in particular human keratin fibres such as the hair, in which the composition according to the invention is applied to said fibres.

Thus, an effective quantity of the composition may be applied to the keratin fibres, followed by an optional rinse after an optional leave-on time.

Preferably, the treatment process comprises a rinsing step after the application of the composition according to the invention.

The invention also relates to the use of said composition for the cosmetic treatment of keratin materials, in particular human keratin fibres such as the hair.

The compositions according to the invention may be used, for example, as a cleaning composition, such as a pre-treatment or posttreatment composition for shampoos, dyes, permanent waves, bleaches and straighteners.

In a preferred manner, the composition according to the invention is a rinse-off or leave-on conditioner care product.

More preferentially, the composition according to the invention is a rinse-off conditioner care product.

The examples that follow illustrate the present invention, and should not in any way be considered as limiting the invention.

### EXAMPLES

In the examples that follow, the amounts are indicated as weight percentages relative to the total weight of the composition (am: active material).

### Example 1

The comparative composition A and the compositions B and C according to the invention are prepared according to the conventional methods.

The percentages are indicated in weight of active material.

| | A (comp.) | B (inv.) | C (inv.) |
|---|---|---|---|
| Jojoba oil | 7.5 | 20 | - |
| Avocado oil | 7.5 | 20 | - |
| Soybean oil | - | - | 40 |
| Mixture of undecane and tridecane (70/30) | 5 | 10 | 10 |
| Fragrance | 0.6 | 0.6 | 06 |
| Glycerol | 3 | 3 | 3 |
| Ethanol | 8 | 8 | 8 |
| PEG-8 isostearate | 4.6 | 7 | - |
| Behenyltrimonium chloride | 2 | 3 | 3 |
| Cetrimonium chloride | - | - | 2 |
| Water | qs 100 | qs 100 | qs 100 |

### Results:

### 1. Stability of the compositions

The compositions B and C according to the invention exhibit good stability over time, better than the comparative composition A, both at ambient temperature and at a temperature of 45°C.

### 2. Deposition of the compounds on the keratin fibres

The compositions are applied to locks of hair pre-washed with a standard shampoo, rinsed and wrung dry.

After a leave-on time, the locks of hair are rinsed and then dried.

The compositions B and C according to the invention make it possible to obtain a greater deposit of the products (fatty substances and cationic surfactants) on the keratin fibres compared with the comparative composition A.

## Claims

1. Cosmetic composition in the form of an oil-in-water nanoemulsion comprising:
(i) at least 40% by weight, relative to the total weight of the composition, of one or more fatty substances including at least one plant oil,
(ii) one or more cationic surfactants chosen from:
- those corresponding to general formula (I) below: 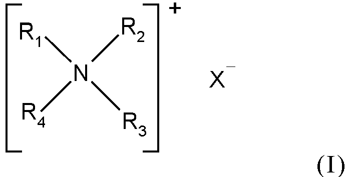
in which the groups R₁ to R₄, which may be identical or different, represent a linear or branched aliphatic group comprising from 1 to 30 carbon atoms, at least one of the groups R₁ to R₄ denoting a linear or branched aliphatic radical comprising from 8 to 30 carbon atoms, preferably from 12 to 24 carbon atoms,
X⁻ is an anion chosen from the group of halides, phosphates, acetates, lactates, (C₁-C₄)alkyl sulfates, (C₁-C₄)alkylsulfonates or (C₁-C₄)alkylarylsulfonates,
- quaternary ammonium salts of imidazoline,
- di- or tri-quaternary ammonium salts,
- quaternary ammonium salts containing at least one ester function, and
(iii) water.

2. Composition according to Claim 1, **characterized in that** the fatty substance(s) are chosen from fatty substances that are liquid at 30°C and at atmospheric pressure, preferably chosen from liquid hydrocarbons, liquid fatty alcohols, liquid fatty esters including plant oils, liquid fatty acids, and mixtures of these compounds.

3. Composition according to Claim 1 or 2, **characterized in that** the plant oil(s) are chosen from one or more triglyceride oils, and in particular chosen from sesame oil, soybean oil, coffee oil, safflower oil, borage oil, sunflower oil, olive oil, apricot kernel oil, camelia oil, bambara pea oil, avocado oil, mango oil, rice bran oil, cottonseed oil, rose oil, kiwi seed oil, sea buckthorn pulp oil, blueberry seed oil, poppy seed oil, orange pip oil, sweet almond oil, palm oil, copra oil, vernonia oil, marjoram oil, baobab oil, rapeseed oil, ximenia oil, pracaxi oil, jojoba oil, shea butter oil, and mixtures thereof.

4. Composition according to any one of the preceding claims, **characterized in that** it comprises, as fatty substance:
- one or more plant oils, in particular chosen from triglyceride oils, preferentially chosen from avocado oil, olive oil, camelia oil, apricot kernel oil, sunflower oil, shea oil, jojoba oil, soybean oil, copra (or coconut) oil, rapeseed oil and mixtures thereof; and
- one or more additional, preferably liquid, fatty substances other than plant oils, better still chosen from linear or branched C₆-C₁₆ alkanes, and in particular chosen from undecane, tridecane, and mixtures thereof.

5. Composition according to any one of the preceding claims, **characterized in that** the fatty substance(s) are present in a total content ranging from 42% to 90% by weight, preferably from 43% to 85% by weight, more preferentially from 45% to 80% by weight, and better still from 48% to 70% by weight, relative to the total weight of the composition.

6. Composition according to any one of the preceding claims, **characterized in that** it comprises at least 30% by weight of one or more plant oils, preferably at least 40% by weight, relative to the total weight of the composition; in particular, the composition comprises the plant oil(s) in a total content ranging from 30% to 90% by weight, preferably from 35% to 85% by weight, more preferentially from 40% to 70% by weight, relative to the total weight of the composition.

7. Composition according to any one of the preceding claims, **characterized in that** the cationic surfactant(s) are chosen from:
- those corresponding to general formula (I) below: 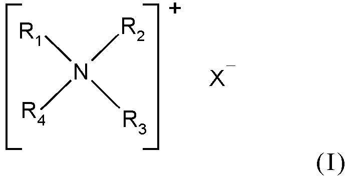
in which the groups R₁ to R₄, which may be identical or different, represent a linear or branched aliphatic group comprising from 1 to 30 carbon atoms, at least one of the groups R₁ to R₄ denoting a linear or branched aliphatic radical comprising from 8 to 30 carbon atoms, preferably from 12 to 24 carbon atoms,
X⁻ is an anion chosen from the group of halides, phosphates, acetates, lactates, (C₁-C₄)alkyl sulfates, (C₁-C₄)alkylsulfonates or (C₁-C₄)alkylarylsulfonates,
- quaternary ammonium salts of imidazoline of formula (II) below: 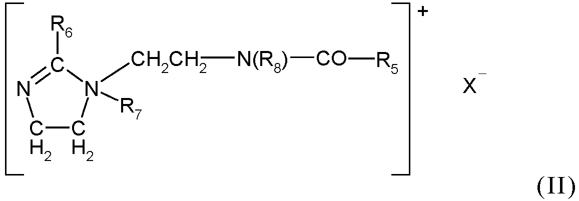 in which:
- R₅ represents an alkenyl or alkyl group comprising from 8 to 30 carbon atoms, for example derived from tallow fatty acids,
- R₆ represents a hydrogen atom, a C₁-C₄ alkyl group or an alkenyl or alkyl group comprising from 8 to 30 carbon atoms,
- R₇ represents a C₁-C₄ alkyl group,
- R₈ represents a hydrogen atom, a C₁-C₄ alkyl group,
- X⁻ is an anion chosen from the group of halides, phosphates, acetates, lactates, alkyl sulfates, alkyl- or alkylarylsulfonates in which the alkyl and aryl groups preferably comprise, respectively, from 1 to 20 carbon atoms and from 6 to 30 carbon atoms,
- di- or tri-quaternary ammonium salts of formula (III): 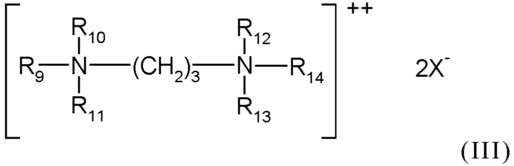 in which:
- R₉ denotes an alkyl radical comprising approximately from 16 to 30 carbon atoms, which is optionally hydroxylated and/or optionally interrupted with one or more oxygen atoms,
- R₁₀ is chosen from hydrogen or an alkyl radical comprising from 1 to 4 carbon atoms or a group (R₉ₐ)(R₁₀ₐ)(R₁₁ₐ)N-(CH₂)₃,
- R₉ₐ, R₁₀ₐ, R₁₁ₐ, R₁₁, R₁₂, R₁₃ and R₁₄, which may be identical or different, are chosen from hydrogen or an alkyl radical comprising from 1 to 4 carbon atoms,
- X⁻ is an anion chosen from the group of halides, acetates, phosphates, nitrates, (C₁-C₄)alkyl sulfates, (C₁-C₄)alkylsulfonates and (C₁-C₄)alkylarylsulfonates, in particular methyl sulfate and ethyl sulfate,
- quaternary ammonium salts containing at least one ester function of formula (IV) below: 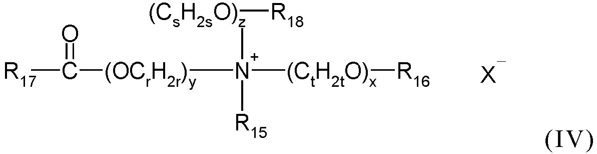 in which:
R₁₅ is chosen from C₁-C₆ alkyl groups and C₁-C₆ hydroxyalkyl or dihydroxyalkyl groups;
R₁₆ is chosen from:
- the group 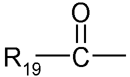
- groups R₂₀, which are linear or branched, saturated or unsaturated C₁-C₂₂ hydrocarbon-based groups,
- a hydrogen atom,
R₁₈ is chosen from:
- the group 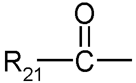
- groups R₂₂, which are linear or branched, saturated or unsaturated C₁-C₆ hydrocarbon-based groups,
- a hydrogen atom,
R₁₇, R₁₉ and R₂₁, which may be identical or different, are chosen from linear or branched, saturated or unsaturated C₇-C₂₁ hydrocarbon-based groups;
r, s and t, which may be identical or different, are integers ranging from 2 to 6;
y is an integer ranging from 1 to 10;
x and z, which may be identical or different, are integers ranging from 0 to 10;
X⁻ is a simple or complex, organic or mineral, anion;
with the proviso that the sum x + y + z is from 1 to 15, that when x is 0 then R₁₆ denotes R₂₀, and that when z is 0 then R₁₈ denotes R₂₂.

8. Composition according to Claim 7, **characterized in that** the cationic surfactant(s) are chosen from those of formula (I) and those of formula (IV), preferably from those of formula (I), more preferentially from behenyltrimethylammonium salts, cetyltrimethylammonium salts, and a mixture of these compounds, and even more preferentially from behenyltrimethylammonium chloride, cetyltrimethylammonium chloride, and a mixture of these compounds.

9. Composition according to any one of the preceding claims, **characterized in that** the cationic surfactant(s) represent from 0.01% to 20% by weight, preferably from 0.1% to 10% by weight and more preferentially from 0.1% to 8% by weight, relative to the total weight of the composition.

10. Composition according to any one of the preceding claims, **characterized in that** it comprises one or more polyols, preferably chosen from polyols comprising at least three carbon atoms and ethylene glycol, more preferentially chosen from 1,3-propanediol, 1,3-butylene glycol, 1,2-pentanediol, dipropylene glycol, hexylene glycol, pentylene glycol, glycerol, ethylene glycol, sorbitol, and a mixture of these compounds, even more preferentially chosen from glycerol, sorbitol or a mixture of these compounds, and in particular the polyol is glycerol.

11. Composition according to Claim 10, **characterized in that** the polyol(s) represent from 1% to 20% by weight, preferably from 2% to 15%, and more preferentially from 3% to 10% by weight, relative to the total weight of the composition.

12. Process for the cosmetic treatment of keratin materials, in particular human keratin fibres such as the hair, in which the composition as defined in any one of the preceding claims is applied to said fibres.

13. Use of the composition as defined in any one of Claims 1 to 11, for the cosmetic treatment of keratin materials, in particular human keratin fibres such as the hair.

## Patentansprüche

1. Kosmetische Zusammensetzung in Form einer Öl-in-Wasser-Nanoemulsion, umfassend:
(i) mindestens 40 Gew.-%, bezogen auf das Gesamtgewicht der Zusammensetzung, einer oder mehrerer Fettsubstanzen einschließlich mindestens eines Pflanzenöls,
(ii) ein oder mehrere kationische Tenside, ausgewählt aus:
- denjenigen der nachstehenden allgemeinen Formel (I): 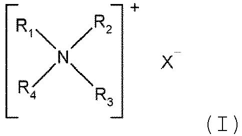
wobei die Gruppen R₁ bis R₄, die gleich oder verschieden sein können, für eine lineare oder verzweigte aliphatische Gruppe mit 1 bis 30 Kohlenstoffatomen stehen, wobei mindestens eine der Gruppen R₁ bis R₄ einen linearen oder verzweigten aliphatischen Rest mit 8 bis 30 Kohlenstoffatomen, vorzugsweise 12 bis 24 Kohlenstoffatomen, bedeutet,
X⁻ ein Anion ist, das aus der Gruppe der Halogenide, Phosphate, Acetate, Lactate, (C₁-C₄)Alkylsulfate, (C₁-C₄)Alkylsulfonate oder (C₁-C₄)Alkylarylsulfonate ausgewählt ist,
- quaternären Ammoniumsalzen von Imidazolin,
- di- oder tri-quaternären Ammoniumsalzen,
- quaternären Ammoniumsalzen mit mindestens einer Esterfunktion und
(iii) Wasser.

2. Zusammensetzung nach Anspruch 1, **dadurch gekennzeichnet, dass** die Fettsubstanz bzw. die Fettsubstanzen aus Fettsubstanzen, die bei 30 °C und Normaldruck flüssig sind, ausgewählt ist bzw. sind und vorzugsweise aus flüssigen Kohlenwasserstoffen, flüssigen Fettalkoholen, flüssigen Fettestern einschließlich Pflanzenölen, flüssigen Fettsäuren und Mischungen dieser Verbindungen ausgewählt ist bzw. sind.

3. Zusammensetzung nach Anspruch 1 oder 2, **dadurch gekennzeichnet, dass** das Pflanzenöl bzw. die Pflanzenöle aus einem oder mehreren Triglyceridölen ausgewählt ist bzw. sind und insbesondere aus Sesamöl, Sojaöl, Kaffeeöl, Safloröl, Borretschöl, Sonnenblumenöl, Olivenöl, Aprikosenkernöl, Kamelienöl, Bambaraerdnussöl, Avocadoöl, Mangoöl, Reiskleieöl, Baumwollsaatöl, Rosenöl, Kiwisamenöl, Sanddornfruchtfleischöl, Heidelbeersamenöl, Mohnsamenöl, Orangenkernöl, Süßmandelöl, Palmöl, Copraöl, Vernoniaöl, Marjoranöl, Baobaböl, Rapsöl, Ximeniaöl, Pracaxiöl, Jojobaöl, Sheabutteröl und Mischungen davon ausgewählt ist bzw. sind.

4. Zusammensetzung nach einem der vorhergehenden Ansprüche, **dadurch gekennzeichnet, dass** sie als Fettsubstanz Folgendes umfasst:
- ein oder mehrere Pflanzenöle, insbesondere ausgewählt aus Triglyceridölen, vorzugsweise ausgewählt aus Avocadoöl, Olivenöl, Kamelienöl, Aprikosenkernöl, Sonnenblumenöl, Sheaöl, Jojobaöl, Sojabohnenöl, Copraöl (oder Kokosnussöl), Rapsöl und Mischungen davon; und
- eine oder mehrere zusätzliche, vorzugsweise flüssige, Fettsubstanzen, die von Pflanzenölen verschieden sind und noch besser aus linearen oder verzweigten C₆-C₁₆-Alkanen und insbesondere aus Undecan, Tridecan und Mischungen davon ausgewählt sind.

5. Zusammensetzung nach dem vorhergehenden Anspruch, **dadurch gekennzeichnet, dass** die Fettsubstanz bzw. die Fettsubstanzen in einer Gesamtmenge im Bereich von 42 bis 90 Gew.-%, vorzugsweise von 43 bis 85 Gew.-%, weiter bevorzugt von 45 bis 80 Gew.-% und noch besser von 48 bis 70 Gew.-%, bezogen auf das Gewicht der Zusammensetzung, vorliegt bzw. vorliegen.

6. Zusammensetzung nach einem der vorhergehenden Ansprüche, **dadurch gekennzeichnet, dass** sie mindestens 30 Gew.-% eines oder mehrerer Pflanzenöle, vorzugsweise mindestens 40 Gew.-%, bezogen auf das Gesamtgewicht der Zusammensetzung, umfasst; insbesondere die Zusammensetzung das Pflanzenöl bzw. die Pflanzenöle in einem Gesamtgehalt im Bereich von 30 bis 90 Gew.-%, vorzugsweise von 35 bis 85 Gew.-%, besonders bevorzugt von 40 bis 70 Gew.-%, bezogen auf das Gesamtgewicht der Zusammensetzung, umfasst.

7. Zusammensetzung nach einem der vorhergehenden Ansprüche, **dadurch gekennzeichnet, dass** das kationische Tensid bzw. die kationischen Tenside aus Folgendem ausgewählt ist bzw. sind:
- denjenigen der nachstehenden allgemeinen Formel (I): 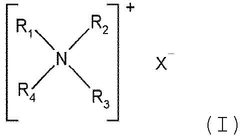
wobei die Gruppen R₁ bis R₄, die gleich oder verschieden sein können, für eine lineare oder verzweigte aliphatische Gruppe mit 1 bis 30 Kohlenstoffatomen stehen, wobei mindestens eine der Gruppen R₁ bis R₄ einen linearen oder verzweigten aliphatischen Rest mit 8 bis 30 Kohlenstoffatomen, vorzugsweise 12 bis 24 Kohlenstoffatomen, bedeutet,
X⁻ ein Anion ist, das aus der Gruppe der Halogenide, Phosphate, Acetate, Lactate, (C₁-C₄)Alkylsulfate, (C₁-C₄)Alkylsulfonate oder (C₁-C₄)Alkylarylsulfonate ausgewählt ist,
- quaternären Ammoniumsalze von Imidazolin der nachstehenden Formel (II): 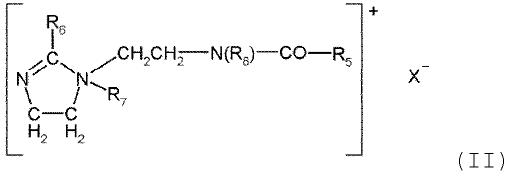 wobei:
- R₅ für eine Alkenyl- oder Alkylgruppe mit 8 bis 30 Kohlenstoffatomen steht, die sich beispielsweise von Talgfettsäuren ableitet,
- R₆ für ein Wasserstoffatom, eine C₁-C₄-Alkylgruppe oder eine Alkenyl- oder Alkylgruppe mit 8 bis 30 Kohlenstoffatomen steht,
- R₇ für eine C₁-C₄-Alkylgruppe steht,
- R₈ für ein Wasserstoffatom oder eine C₁-C₄-Alkylgruppe steht,
- X⁻ ein Anion ist, das aus der Gruppe der Halogenide, Phosphate, Acetate, Lactate, Alkylsulfate, Alkyl- oder Alkylarylsulfonate, in denen die Alkyl- und Arylgruppen vorzugsweise 1 bis 20 Kohlenstoffatome und 6 bis 30 Kohlenstoffatome umfassen, ausgewählt ist,
- di- oder triquaternären Ammoniumsalzen der Formel (III): 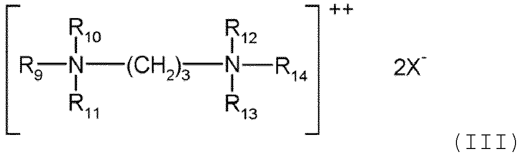 wobei:
- R₉ einen Alkylrest mit ungefähr 16 bis 30 Kohlenstoffatomen, der gegebenenfalls hydroxyliert und/oder gegebenenfalls durch ein oder mehrere Sauerstoffatome unterbrochen ist, bedeutet,
- R₁₀ aus Wasserstoff oder einem Alkylrest mit 1 bis 4 Kohlenstoffatomen oder einer Gruppe (R₉ₐ) (R₁₀ₐ) (R₁₁ₐ)N-(CH₂)₃ ausgewählt ist,
- R₉ₐ, R₁₀ₐ, R₁₁ₐ, R₁₁, R₁₂, R₁₃ und R₁₄, die gleich oder verschieden sein können, aus Wasserstoff oder einem Alkylrest mit 1 bis 4 Kohlenstoffatomen ausgewählt sind,
- X⁻ ein Anion ist, das aus der Gruppe der Halogenide, Acetate, Phosphate, Nitrate, (C₁-C₄)Alkylsulfate, (C₁-C₄)Alkylsulfonate und (C₁-C₄)Alkylarylsulfonate, insbesondere Methylsulfat und Ethylsulfat, ausgewählt ist,
- quaternären Ammoniumsalzen mit mindestens einer Esterfunktion der nachstehenden Formel (IV): 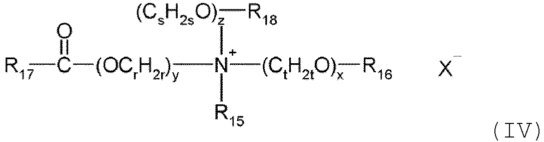 wobei:
R₁₅ aus C₁-C₆-Alkylgruppen und C₁-C₆-Hydroxyalkyl- oder - Dihydroxyalkylgruppen ausgewählt ist;
R₁₆ ausgewählt ist aus: 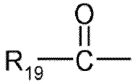
- der Gruppe
- Gruppen R₂₀, bei denen es sich um lineare oder verzweigte, gesättigte oder ungesättigte C₁-C₂₂-Gruppen auf Kohlenwasserstoffbasis handelt,
- einem Wasserstoffatom,
R₁₈ ausgewählt ist aus: 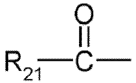
- der Gruppe
- Gruppen R₂₂, bei denen es sich um lineare oder verzweigte, gesättigte oder ungesättigte C₁-C₆-Gruppen auf Kohlenwasserstoffbasis handelt,
- einem Wasserstoffatom,
R₁₇, R₁₉ und R₂₁, die gleich oder verschieden sein können, aus linearen oder verzweigten, gesättigten oder ungesättigten C₇-C₂₁-Gruppen auf Kohlenwasserstoffbasis ausgewählt sind;
r, s und t, die gleich oder verschieden sein können, ganze Zahlen im Bereich von 2 bis 6 sind;
y eine ganze Zahl im Bereich von 1 bis 10 ist;
x und z, die gleich oder verschieden sein können, ganze Zahlen im Bereich von 0 bis 10 sind;
X⁻ ein einfaches oder komplexes, organisches oder mineralisches Anion ist;
mit der Maßgabe, dass die Summe x + y + z 1 bis 15 beträgt, dass dann, wenn x 0 ist, R₁₆ R₂₀ bedeutet und dass dann, wenn z 0 ist, R₁₈ R₂₂ bedeutet.

8. Zusammensetzung nach Anspruch 7, **dadurch gekennzeichnet, dass** das kationische Tensid bzw. die kationischen Tenside aus denjenigen der Formel (I) und denjenigen der Formel (IV), vorzugsweise aus denjenigen der Formel (I), weiter bevorzugt aus Behenyltrimethylammoniumsalzen, Cetyltrimethylammoniumsalzen und einer Mischung dieser Verbindungen, und noch weiter bevorzugt aus Behenyltrimethylammoniumchlorid, Cetyltrimethylammoniumchlorid und eine Mischung dieser Verbindungen ausgewählt ist bzw. sind.

9. Zusammensetzung nach einem der vorhergehenden Ansprüche, **dadurch gekennzeichnet, dass** das kationische Tensid bzw. die kationischen Tenside in einer Menge von 0,01 bis 20 Gew.-%, vorzugsweise von 0,1 bis 10 Gew.-% und weiter bevorzugt von 0,1 bis 8 Gew.-%, bezogen auf das Gesamtgewicht der Zusammensetzung, vorliegt bzw. vorliegen.

10. Zusammensetzung nach einem der vorhergehenden Ansprüche, **dadurch gekennzeichnet, dass** sie ein oder mehrere Polyole umfasst, die vorzugsweise aus Polyolen mit mindestens drei Kohlenstoffatomen und Ethylenglykol, weiter bevorzugt aus 1,3-Propandiol, 1,3-Butylenglykol, 1,2-Pentandiol, Dipropylenglykol, Hexylenglykol, Pentylenglykol, Glycerin, Ethylenglykol, Sorbitol und einer Mischung dieser Verbindungen und noch weiter bevorzugt aus Glycerin, Sorbitol oder einer Mischung dieser Verbindungen ausgewählt sind, und es sich bei dem Polyol insbesondere um Glycerin handelt.

11. Zusammensetzung nach Anspruch 10, **dadurch gekennzeichnet, dass** das Polyol bzw. die Polyole 1 bis 20 Gew.-%, vorzugsweise 2 bis 15 Gew.-% und weiter bevorzugt 3 bis 10 Gew.-%, bezogen auf das Gesamtgewicht der Zusammensetzung, ausmacht bzw. ausmachen.

12. Verfahren zur kosmetischen Behandlung von Keratinmaterialien, insbesondere menschlichen Keratinfasern wie dem Haar, bei dem die Zusammensetzung gemäß einem der vorhergehenden Ansprüche auf diese Fasern aufgebracht wird.

13. Verwendung der Zusammensetzung gemäß einem der Ansprüche 1 bis 11 zur kosmetischen Behandlung von Keratinmaterialien, insbesondere menschlichen Keratinfasern wie dem Haar.

## Revendications

1. Composition cosmétique sous forme de nanoémulsion huile-dans-eau comprenant :
(i) au moins 40 % en poids, par rapport au poids total de la composition, d'un ou plusieurs corps gras dont au moins une huile végétale,
(ii) un ou plusieurs tensioactifs cationiques choisis parmi :
- ceux répondant à la formule générale (I) suivante : 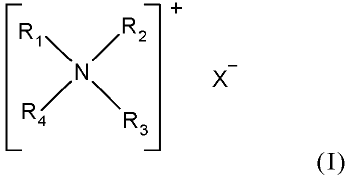
dans laquelle les groupes R₁ à R₄, qui peuvent être identiques ou différents, représentent un groupe aliphatique, linéaire ou ramifié, comportant de 1 à 30 atomes de carbone, au moins un des groupes R₁ à R₄ désignant un radical aliphatique, linéaire ou ramifié, comportant de 8 à 30 atomes de carbone, de préférence de 12 à 24 atomes de carbone,
X⁻ est un anion choisi dans le groupe des halogénures, phosphates, acétates, lactates, alkyl(C₁-C₄)sulfates, alkyl(C₁-C₄)sulfonates ou alkyl(C₁-C₄)aryl-sulfonates,
- les sels d'ammonium quaternaire de l'imidazoline,
- les sels de di- ou tri-ammonium quaternaire,
- les sels d'ammonium quaternaire contenant au moins une fonction ester, et
(iii) de l'eau.

2. Composition selon la revendication 1, **caractérisée en ce que** le ou les corps gras sont choisis parmi les corps gras liquides à 30°C et à pression atmosphérique, de préférence choisis parmi les hydrocarbures liquides, les alcools gras liquides, les esters gras liquides dont les huiles végétales, les acides gras liquides et les mélanges de ces composés.

3. Composition selon la revendication 1 ou 2, **caractérisée en ce que** le ou les huiles végétales sont choisis parmi une ou plusieurs huiles triglycérides, et en particulier choisies parmi les huiles de sésame, de soja, de café, de carthame, de bourrache, de tournesol, d'olive, d'amande d'abricot, de camélia, de pois bambara, d'avocat, de mangue, de son de riz, de coton, de rosier, de pépins de kiwi, de pulpe d'argousier, de pépins de myrtille, de pavot, de pépins d'orange, d'amande douce, de palme, de coprah, de vernonia, de marjolaine, de baobab, de colza, de ximénia, de pracaxi, l'huile de jojoba, l'huile de beurre de karité, et leurs mélanges.

4. Composition selon l'une quelconque des revendications précédentes, **caractérisée en ce qu'**elle comprend, à titre de corps gras :
- une ou plusieurs huiles végétales, en particulier choisies parmi les huiles triglycérides, préférentiellement parmi l'huile d'avocat, l'huile d'olive, l'huile de camélia, l'huile d'amande d'abricot, l'huile de tournesol, l'huile de karité, l'huile de jojoba, l'huile de soja, l'huile de coprah (ou coco), l'huile de colza et leurs mélanges ; et
- un ou plusieurs corps gras additionnels autres que les huiles végétales, de préférence liquides, mieux choisis parmi les alcanes en C₆-C₁₆ linéaires ou ramifiés, et en particulier choisis parmi l'undécane, le tridécane, et leurs mélanges.

5. Composition selon l'une quelconque des revendications précédentes, **caractérisée en ce que** le ou corps gras sont présents dans une teneur totale allant de 42% à 90 % en poids, de préférence de 43% à 85 % en poids, plus préférentiellement de 45% à 80% en poids, et mieux de 48% à 70 % en poids, par rapport au poids total de la composition.

6. Composition selon l'une quelconque des revendications précédentes, **caractérisée en ce qu'**elle comprend au moins 30% en poids d'une ou plusieurs huiles végétales, de préférence au moins 40% en poids, par rapport au poids total de la composition ; en particulier la composition comprend le ou les huiles végétales en une teneur totale allant de 30% à 90% en poids, de préférence de 35% à 85% en poids, plus préférentiellement de 40% à 70% en poids par rapport au poids total de la composition.

7. Composition selon l'une quelconque des revendications précédentes **caractérisée en ce que** le ou les tensioactifs cationiques sont choisis parmi :
- ceux répondant à la formule générale (I) suivante : 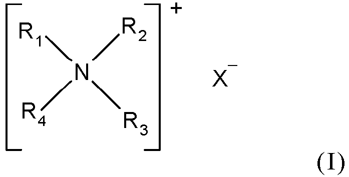
dans laquelle les groupes R₁ à R₄, qui peuvent être identiques ou différents, représentent un groupe aliphatique, linéaire ou ramifié, comportant de 1 à 30 atomes de carbone, au moins un des groupes R₁ à R₄ désignant un radical aliphatique, linéaire ou ramifié, comportant de 8 à 30 atomes de carbone, de préférence de 12 à 24 atomes de carbone,
X⁻ est un anion choisi dans le groupe des halogénures, phosphates, acétates, lactates, alkyl(C₁-C₄)sulfates, alkyl(C₁-C₄)sulfonates ou alkyl(C₁-C₄)aryl-sulfonates,
- les sels d'ammonium quaternaire de l'imidazoline de formule (II) suivante : 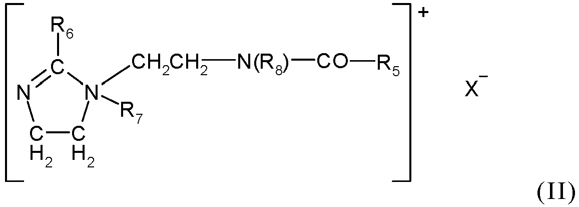 dans laquelle :
- R₅ représente un groupe alcényle ou alkyle comportant de 8 à 30 atomes de carbone, par exemple dérivés des acides gras du suif,
- R₆ représente un atome d'hydrogène, un groupe alkyle en C₁-C₄ ou un groupe alcényle ou alkyle comportant de 8 à 30 atomes de carbone,
- R₇ représente un groupe alkyle en C₁-C₄,
- R₈ représente un atome d'hydrogène, un groupe alkyle en C₁-C₄,
- X⁻ est un anion choisi dans le groupe des halogénures, phosphates, acétates, lactates, alkylsulfates, alkyl- ou alkylarylsulfonates dont les groupements alkyl et aryl comprennent de préférence respectivement de 1 à 20 atomes de carbone et de 6 à 30 atomes de carbone,
- les sels de di ou de triammonium quaternaire de formule (III) : 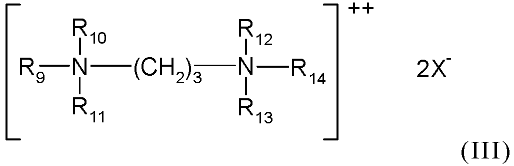 dans laquelle :
- R₉ désigne un radical alkyle comportant environ de 16 à 30 atomes de carbone éventuellement hydroxylé et/ou éventuellement interrompu par un ou plusieurs atomes d'oxygène,
- R₁₀ est choisi parmi l'hydrogène ou un radical alkyle comportant de 1 à 4 atomes de carbone ou un groupe (R₉ₐ)(R₁₀ₐ)(R₁₁ₐ)N-(CH₂)₃,
- R₉ₐ, R₁₀ₐ, R₁₁ₐ, R₁₁, R₁₂, R₁₃ et R₁₄, identiques ou différents, sont choisis parmi l'hydrogène ou un radical alkyle comportant de 1 à 4 atomes de carbone,
- X⁻ est un anion choisi dans le groupe des halogénures, acétates, phosphates, nitrates alkyl(C₁-C₄)sulfates, alkyl(C₁-C₄)sulfonates et alkyl(C₁-C₄)aryl-sulfonates, en particulier méthylsulfate et éthylsulfate,
- les sels d'ammonium quaternaire contenant au moins une fonction ester de formule (IV) suivante : 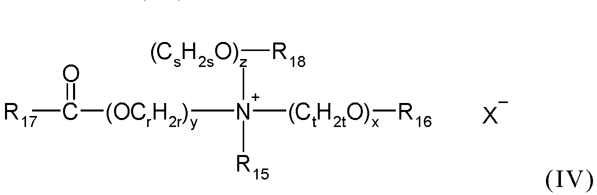 dans laquelle :
R₁₅ est choisi parmi les groupes alkyles en C₁-C₆ et les groupes hydroxyalkyles ou dihydroxyalkyles en C₁-C₆ ;
R₁₆ est choisi parmi :
- le groupe 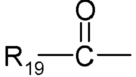
- les groupes R₂₀ qui sont des groupes hydrocarbonés en C₁-C₂₂, linéaires ou ramifiés, saturés ou insaturés,
- l'atome d'hydrogène,
R₁₈ est choisi parmi :
- le groupe 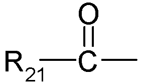
- les groupes R₂₂ qui sont des groupes hydrocarbonés en C₁-C₆, linéaires ou ramifiés, saturés ou insaturés,
- l'atome d'hydrogène,
R₁₇, R₁₉ et R₂₁, identiques ou différents, sont choisis parmi les groupes hydrocarbonés en C₇-C₂₁, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ;
X⁻ est un anion simple ou complexe, organique ou inorganique ;
sous réserve que la somme x + y + z vaut de 1 à 15, que lorsque x vaut 0 alors R₁₆ désigne R₂₀ et que lorsque z vaut 0 alors R₁₈ désigne R22.

8. Composition selon la revendication 7, **caractérisée en ce que** le ou les tensioactifs cationiques sont choisis parmi ceux de formule (I) et ceux de formule (IV), de préférence parmi ceux de formule (I), plus préférentiellement parmi les sels de béhényltriméthylammonium, les sels de cétyltriméthylammonium, et un mélange de ces composés, et plus préférentiellement encore parmi le chlorure de behényltriméthylammonium, le chlorure de cétyltriméthylammonium, et un mélange de ces composés.

9. Composition selon l'une quelconque des revendications précédentes, **caractérisée en ce que** le ou les tensioactifs cationiques représentent de 0,01% à 20 % en poids, de préférence de 0,1% à 10 % en poids, et plus préférentiellement de 0,1% à 8 % en poids, par rapport au poids total de la composition.

10. Composition selon l'une quelconque des revendications précédentes, **caractérisée en ce qu'**elle comprend un ou plusieurs polyols, de préférence choisis parmi les polyols comprenant au moins trois atomes de carbone et l'éthylène glycol, plus préférentiellement choisis parmi le 1,3-propanediol, le 1,3-butylène glycol, le pentane-1,2-diol, le dipropylène glycol, l'hexylène glycol, le pentylène glycol, le glycérol, l'éthylène glycol, le sorbitol, et un mélange de ces composés, encore plus préférentiellement choisis parmi le glycérol, le sorbitol ou un mélange de ces composés, et en particulier le polyol est le glycérol.

11. Composition selon la revendication 10, **caractérisée en ce que** le ou les polyols représentent de 1% à 20 % en poids, de préférence de 2% à 15% en poids, et plus préférentiellement de 3% à 10 % en poids, par rapport au poids total de la composition.

12. Procédé de traitement cosmétique des matières kératiniques, en particulier des fibres kératiniques humaines telles que les cheveux, dans lequel la composition telle que définie dans l'une quelconque des revendications précédentes est appliquée sur lesdites fibres.

13. Utilisation de la composition telle que définie selon l'une quelconque des revendications 1 à 11, pour le traitement cosmétique des matières kératiniques, en particulier des fibres kératiniques humaines telles que les cheveux.
